# EUROPEAN PATENT SPECIFICATION

(11) **EP 2 057 931 B1**
(45) Date of publication and mention of the grant of the patent: **09.01.2013**
(21) Application number: 07793025.3
(22) Date of filing: 27.08.2007
(51) Int. Cl.: A61B 1/04, A61B 1/00, A61B 5/07, A61B 5/06

(54) **Capsule endoscope guiding system**
Führungssystem für Kapselendoskope
Système de guidage de capsule endoscopique

(30) Priority: 29.08.2006 JP 2006232789
(43) Date of publication of application: 13.05.2009
(73) Proprietor: Olympus Medical Systems Corp., Tokyo 151-0072 (JP); Olympus Corporation, Tokyo 151-0072 (JP)
(72) Inventor: UCHIYAMA, Akio, Tokyo 151-0072 (JP); SATO, Ryoji, Tokyo 151-0072 (JP); KIMURA, Atsushi, Tokyo 151-0072 (JP); CHIBA, Atsushi, Tokyo 151-0072 (JP); TAKIZAWA, Hironobu, Tokyo 151-0072 (JP); MORI, Takeshi, Tokyo 151-0072 (JP); MINAI, Tetsuo, Tokyo 151-0072 (JP)
(74) Representative: von Hellfeld, Axel
(86) International application number: PCT/JP2007/066573
(87) International publication number: WO 2008/026549

(56) References cited:
- WO-A1-2006/014011
- JP-A- 2005 004 383
- JP-A- 2005 004 383
- JP-A- 2005 304 638
- JP-A- 2006 513 001
- US-A1- 2004 210 131
- US-A1- 2006 173 265

## Description

### TECHNICAL FIELD

The present invention relates to a capsule guiding system which allows a communication with a capsule endoscope via a human body communication and a guidance of the capsule endoscope through a detection of at least one of a position and a direction of the capsule endoscope.

### BACKGROUND ART

Recently, a swallowable capsule endoscope has appeared in the field of endoscopes. This capsule endoscope is provided with an imaging function and a radio function. The capsule endoscope moves inside a body cavity, for example, inside of organs such as the stomach and the small intestine according to the peristaltic movement thereof, and functions to capture images sequentially during a period which starts when the capsule endoscope is swallowed from a mouth of a patient for the purpose of an observation (examination) and ends when it is naturally excreted from a human body.

However, due to a communication with an outside of the human body through the radio function, the capsule endoscope has problems that a large power consumption causes a short operation period, and a large capacity occupied by a primary battery prevents downsizing and enhancing a high performance of the capsule endoscope. In response, a capsule endoscope which performs the communication with the outside of the human body through a human body communication has been proposed recently. In the capsule endoscope using the human body communication, an electric current is generated due to a potential difference between transmitting electrodes formed on a surface of the capsule endoscope, a voltage is induced between two receiving electrodes attached on a surface of the human body when the electric current flows through the human body, and data is received from the capsule endoscope via the induced voltage. Since the capsule endoscope using the human body communication does not need a high-frequency signal of several hundred MHz and can transmit data by a low-frequency signal of about 10 MHz, the consumption of the electric power can be enormously reduced (see Patent Documents 1 and 2).

On the other hand, there is a system in which a capsule endoscope is provided with a magnet and subjected to an external rotating magnetic field, so that the capsule endoscope is rotated, and this rotation allows guiding the capsule endoscope in a subject body to a desired position and performing an examination (see Patent Documents 3 and 4).
Patent Document 1: Japanese translation No. 2006-513001 of PCT international application
Patent Document 2: Japanese translation No. 2006-513670 of PCT international application
Patent Document 3: Japanese Patent Application Laid-Open No. 2004-255174
Patent Document 4: Japanese Patent Application Laid-Open No. 2005-304638

WO 2006/014011 discloses a medical apparatus having an insertion unit inserted into a body cavity, wherein a position-posture detecting unit detects at least one of the position and posture of the insertion unit. Magnetic field generating units are arranged in an axis-symmetrical manner on the plane. The position-posture detecting unit communicates with a position posture varying unit which moves a bed on which a patient is disposed, or moves the magnetic field generating unit, such that the central axis of the magnetic field of the insertion unit corresponds to a magnetic field generated by the magnetic field generating unit. A magnetic field operated unit of the insertion unit is operated by the magnetic field generating unit to control the orientation of the insertion unit within the bodily cavity. The magnetic field generating unit is disposed beneath the bed of the patient.

US 2004/210131 discloses a system for measuring the position of an in vivo radio device, comprising an in vivo radio device administered into a living organism, and plurality of ex vivo radio devices disposed outside of the living organism, and a position measuring device. The position measuring unit measures the position of the in vivo radio device using the receiving characteristics of the position measuring signal received by the ex vivo radio devices.

US 2006/173265 discloses an in vivo device and a plurality of receiver electrodes disposed on a surface of the human body, wherein a position of the in vivo device is determined based on the strength of the received signal from the electrodes disposed on the human body.

JP 2005/004383 discloses an electric field use monitoring system comprising a plurality of electrodes provided on a floor and a mobile terminal having a transceiver using an electric field induced in the human body, and an electric field based upon a signal to be detected is induced in the body of a person who walks on the floor, is detected by a small-sized information terminal, and is outputted as a detection electric signal, which analyzes the detection signal to decide the state of the person.

### DISCLOSURE OF INVENTION

### PROBLEM TO BE SOLVED BY THE INVENTION

However, when it is intended to apply the system of using the external rotating magnetic field and guiding the capsule endoscope to the human body communication system described above, there are problems that a positional relationship between the transmitting electrodes of the capsule endoscope and the receiving electrodes would easily change due to a movement of the human body, at least one of a position and a direction of the capsule endoscope in the subject body cannot be detected precisely, and the guiding cannot be performed precisely as a result.

The present invention has been achieved in view of the foregoing and an object of the present invention is to provide a capsule guiding system which allows a precise detection of at least one of a position and a direction, inside a subject body, of a capsule endoscope using a human body communication, and a precise guidance of the capsule endoscope.

### MEANS FOR SOLVING PROBLEM

To solve the problems described above and achieve the object, a capsule guiding system according to the present invention includes the features of claim 1.

### EFFECT OF THE INVENTION

In the capsule guiding system according to the present invention, since the detector detects the relative position between the electrode pad and the magnetically guiding device; and the calculator calculates at least one of the position and the direction of the capsule endoscope based on the detection value of the electrode pad and calculates at least one of the absolute position and the absolute direction of the capsule endoscope with respect to the magnetically guiding device based on at least one of the position and the direction of the capsule endoscope and on the relative position detected by the detector, at least one of the absolute position and the absolute direction of the capsule endoscope can be detected precisely and the capsule endoscope can be guided precisely as a result even in the case of performing the human body communication.

Besides, in the capsule guiding system according to the present invention, since the electrode pad is fixedly arranged on the rigid part of the human body arranging device; and the calculator calculates at least one of the position and the direction of the capsule endoscope based on the detection value of the electrode pad, adds at least one of the position and the direction of the rigid part of the human body arranging device with respect to the magnetically guiding device to at least one of the position and the direction of the capsule endoscope, and calculates at least one of the absolute position and the absolute direction of the capsule endoscope with respect to the magnetically guiding device, at least one of the absolute position and the absolute direction of the capsule endoscope can be detected precisely with a simple configuration and the capsule endoscope can be guided precisely as a result even in the case of performing the human body communication.

Moreover, in a capsule guiding method, since at least one of the relative position and the relative direction of the capsule endoscope with respect to the electrode pad which performs the human body communication with the capsule endoscope inside the human body to receive a human body communication signal is calculated based on the human body communication signal; the relative position of the electrode pad with respect to the magnetically guiding device which forms an external magnetic field with respect to the capsule endoscope and magnetically guides the capsule endoscope is calculated; at least one of the absolute position and the absolute direction of the capsule endoscope with respect to the magnetically guiding device is calculated based on at least one of the relative position and the relative direction of the capsule endoscope and on the relative position of the electrode pad; and the magnetically guiding device is controlled based on at least one of the absolute position and the absolute direction of the capsule endoscope, at least one of the absolute position and the absolute direction of the capsule endoscope can be detected precisely and the capsule endoscope can be guided precisely as a result even in the case of performing the human body communication.

### BRIEF DESCRIPTION OF DRAWINGS

FIG. 1 is a schematic view showing a structure of a capsule guiding system according to a first embodiment of the present invention.
FIG. 2 is a view showing a structure of a capsule endoscope in the capsule guiding system shown in FIG. 1.
FIG. 3 is a view showing a structure of a magnetic field generating device in the capsule guiding system shown in FIG. 1.
FIG. 4 is a schematic view showing a structure of a capsule guiding system according to a modification of the first embodiment not forming part of the present invention.
FIG. 5 is a schematic view showing a structure of a capsule guiding system according to a second embodiment not forming part of the present invention.
FIG. 6 is a cross sectional view of a modification using a sheet member.
FIG. 7 is a schematic view of a structure of a capsule guiding system according to a third embodiment not forming part of the present invention.
FIG. 8 is a schematic view of a structure of a capsule guiding system according to a fourth embodiment not forming part of the present invention.
FIG. 9 shows a specific example of a marker shown in FIG. 8.

### EXPLANATIONS OF LETTERS OR NUMERALS

- 1: Magnetic field generating device
- 2: Base
- 3: Bed
- 4: Conductive gel bed
- 5: Human body
- 6: Capsule endoscope
- 7, 67, 77: Electrode pad
- 10, 50, 60, 70: Capsule guiding system
- 11: Receiver
- 12: Position/direction calculator
- 13: Control unit
- 15: Display unit
- 16: Input unit
- 17: Storage unit
- 18, 74: Image processor
- 19: Guiding magnetic field controller
- 20: Tubular case
- 21: Dome-shaped case
- 22, 23: Transmitting electrode
- 24: Spiral protrusion
- 43: Bathtub
- 44: Conductive fluid
- 51: Selector
- 57: Pressure sensor
- 61: Sensor
- 71: Marker
- 72, 73: Imaging device

### BEST MODE(S) FOR CARRYING OUT THE INVENTION

Exemplary embodiments of a capsule guiding system and a capsule guiding method will be explained below with reference to the accompanying drawings. It should be noted that the present invention is not limited by those embodiment but by the appended claims.

### First embodiment

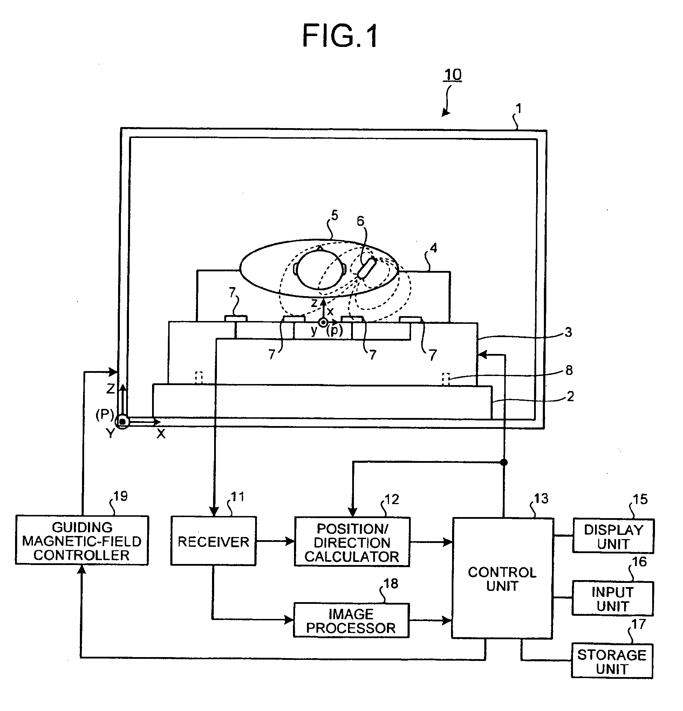

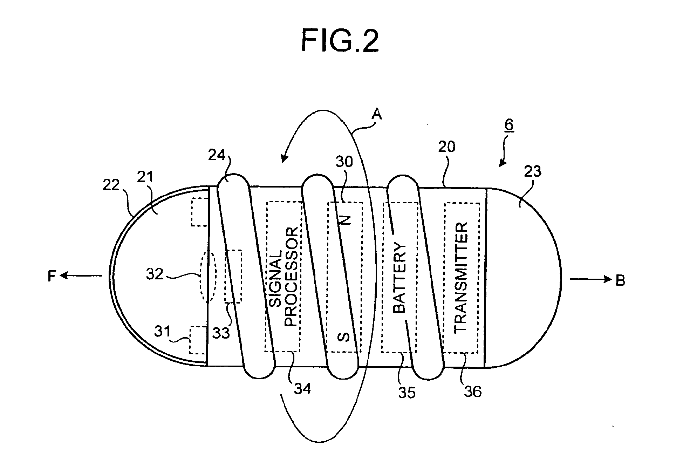

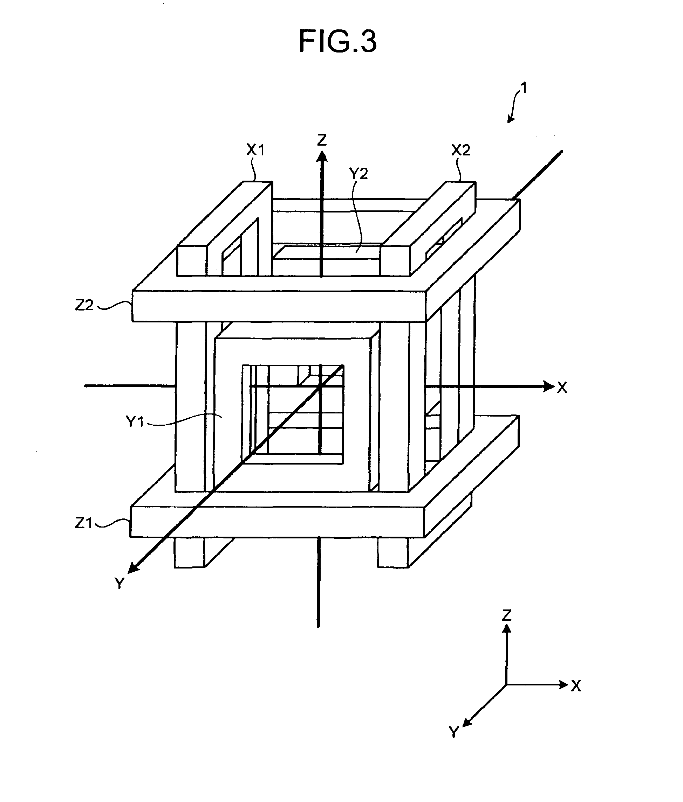

FIG. 1 is a schematic view showing a structure of a capsule guiding system according to a first embodiment of the present invention. FIG. 2 is a view showing a structure of a capsule endoscope shown in FIG. 1. In addition, FIG. 3 is a view showing a structure of a magnetic field generating device shown in FIG. 1. In FIGS. 1 to 3, a capsule guiding system 10 includes: a magnetic field generating device 1 which generates a three-dimensional rotating magnetic field; a base 2 at least one part of which is provided in the magnetic field generating device 1; a bed 3 (a rigid part of a human body arranging device) which is movable in a Y direction on an upper part of the base 2 via a guide 8 and serves to arrange a human body 5; a plurality of electrode pads 7 which are fixedly arranged like a matrix on an upper part of the bed 3; and a conductive gel bed 4 (a conductive soft part of the human body arranging device) which is arranged on the bed 3 and the electrode pads 7 and formed of a soft member having a conductivity, for example, a conductive gel member. The human body 5 as a subject body has a capsule endoscope 6 which is swallowed from a mouth and can perform a human body communication, and becomes a state of being electrically conducted with the conductive gel member by lying down on the conductive gel bed 4. Here, the state of being electrically conducted with the conductive gel bed 4 is maintained at least in a part of the human body 5 and positions of the electrode pads 7 do not move even when the human body 5 moves. Here, a conductive rubber and the like may be used instead of the conductive gel member. Besides, though the electrode pads are configured to be fixedly arranged like a matrix, the electrode pads may not be arranged necessarily like a matrix and it is only necessary that they are fixed at given positions, respectively.

Each of the electrode pads 7 is connected to a receiver 11 which receives voltages induced in the electrode pads 7 and outputs to a position/direction calculator 12 and an image processor 18 as a reception signal transmitted from the capsule endoscope 6 via the human body 5. The position/direction calculator 12 calculates at least one of a relative position and a relative direction of the capsule endoscope 6 with respect to the bed 3 based on the voltage values induced in the electrode pads 7. On the other hand, the image processor 18 generates image information transmitted from the capsule endoscope 6 based on the reception signal output from the receiver 11 and outputs to a control unit 13.

The control unit 13 is connected to a display unit 15, an input unit 16, and a storage unit 17, and makes the display unit 15 display and the storage unit 17 sequentially store the image information input from the image processor 18. The input unit 16 outputs input information including various operations with respect to the magnetic field generating device 1 to the control unit 13, and the control unit 13 gives an instruction to a guiding magnetic field controller 19 and controls a movement of the bed 3 with respect to the base 2 based on the input information. Information for controlling the movement of the bed 3 is also input to the position/direction calculator 12. The position/direction calculator 12 corrects a movement amount of the bed 3 obtained from the information for controlling the movement of the bed 3 based on a reference position p of the bed 3 with respect to a reference position P of the magnetic field generating device 1, finally calculates at least one of an absolute position and an absolute direction of the capsule endoscope 6 seen from the reference position P of the magnetic field generating device 1 based on a value of at least one of the relative position and the relative direction, which are described above, of the capsule endoscope 6 seen from the reference position p of the bed 3, and transmits the calculated result to the control unit 13. The control unit 13 transmits the value of at least one of the absolute position and the absolute direction of the capsule endoscope 6 to the guiding magnetic field controller 19, makes the storage unit 17 temporarily store therein, and uses the value in displaying the position/direction of the capsule endoscope 6 on the display unit 15.

The capsule endoscope 6 is shaped to have an opaque tubular case 20 whose one end has an opaque dome shape and the other end is blocked with a transparent dome-shaped case 21 as shown in FIG. 2. In an inside of the tubular case 20 and the dome-shaped case 21, an illumination unit 31 realized by a light emitting diode and the like, a focusing lens 32, and an imaging element 33 are provided at a side of the dome-shaped case 21 and an image of a subject around the side of the dome-shaped case 21 is captured. An imaging signal output from the imaging element 33 is processed by a signal processor 34, output as an image signal from transmitting electrodes 22 and 23 to be described later via a transmitter 36, and transmitted to the electrode pads 7 through the human body. Here, the transmitting electrodes 22 and 23 used for the human body communication are respectively formed on a surface of the dome-shaped case 21 and a surface of the dome opposite to the dome-shaped case 21. The transmitting electrode 22 formed on the surface of the dome-shaped case 21 is a transparent electrode realized by an indium tin oxide and the like. Besides, each of the transmitting electrodes 22 and 23 is a metal which is safe to the human body and has good corrosion resistance, and the transmitting electrode 23 is realized by SUS316L, gold, and the like for example. Moreover, the transmitting electrodes 22 and 23 are to be electrically connected to an inside of the human body via a body fluid and the like.

A battery 35 and a magnet 30 are arranged at a center part of the capsule endoscope 6. Magnetic poles of the magnet 30 are arranged in a direction perpendicular to a longitudinal direction, that is, an axial direction of the capsule endoscope 6 and when a rotating magnetic field is applied around the axis, the magnet 30 is drawn and rotated around the axis like a rotor of a motor, so that the capsule endoscope 6 is rotated. Here, a spiral protrusion 24 is formed around a cylindrical part of the capsule endoscope 6 and the capsule endoscope 6 moves in the axial direction like a screw since the spiral protrusion 24 serves to screw together with a wall of a digestive tract inside the body when the capsule endoscope 6 is rotated. For example, when the capsule endoscope 6 is rotated in a direction A around the axis, the capsule endoscope 6 moves forward towards a direction F, and when the capsule endoscope 6 is rotated in a reverse direction to the direction A around the axis, the capsule endoscope 6 moves backward towards a direction B in FIG. 2. By this, the capsule endoscope 6 can move inside the body according to the rotating/guiding magnetic field of the magnetic field generating device 1.

Besides, as shown in FIG. 3, the magnetic field generating device 1 has a configuration in which three pairs of electromagnets, each electromagnet producing a state where a coil is wound around a member such as a ferromagnet having a high dielectric constant, are fitted together respectively in three directions X, Y, and Z so that the human body 5 is put thereamong, and can form a three-dimensional external rotating magnetic field with respect to the capsule endoscope 6 by controlling a strength of the magnetic field generated in each direction. The external rotating magnetic field can be formed when the guiding magnetic field controller 19 controls a power distribution amount to each electromagnet pair of each direction based on an operational instruction from the input unit 16 via the control unit 13.

Next, a capsule guiding method in which the capsule guiding system 10 having the above-described configuration uses the rotating magnetic field to guide the capsule endoscope 6 inside the human body 5 will be explained. First, the position/direction calculator 12 obtains, from each electrode pad 7, a human body communication signal as a reception signal transmitted via the human body 5 from the capsule endoscope 6 inside the human body 5 and calculates at least one of a relative position and a relative direction of the capsule endoscope 6 with respect to each electrode pad 7 based on a voltage value of the human body communication signal from each electrode pad 7, that is, voltage values induced in the electrode pads 7 (a capsule position/direction calculating step). Here, when the electrode pads 7 are fixedly arranged on the bed 3, the position/direction calculator 12 calculates at least one of the relative position and the relative direction of the capsule endoscope 6 with respect to the bed 3 in the capsule position/direction calculating step as described above.

Subsequently, the position/direction calculator 12 calculates a relative position of each electrode pad 7 with respect to the magnetic field generating device 1 (an electrode pad position calculating step). In this case, the position/direction calculator 12 calculates the relative position of each electrode pad 7 with respect to the reference position P of the magnetic field generating device 1 based on the reference position p of the bed 3 with respect to the reference position P of the magnetic field generating device 1 and on a fixed position of each electrode pad 7 on the bed 3, and corrects the calculated relative position of each electrode pad 7 based on the movement amount of the bed 3 described above. When a relative positional relationship between each electrode pad 7 and the bed 3 is always constant, the relative position of each electrode pad 7 with respect to the reference position P of the magnetic field generating device 1 may be set in advance based on the relative positional relationship between the reference position P of the magnetic field generating device 1 and the reference position p of the bed 3.

Next, the position/direction calculator 12 calculates at least one of an absolute position and an absolute direction of the capsule endoscope 6 seen from the reference position P of the magnetic field generating device 1 based on at least one of the relative position and the relative direction of the capsule endoscope 6 with respect to each electrode pad 7 and the relative position of each electrode pad 7 with respect to the reference position P of the magnetic field generating device 1 (an absolute position/direction calculating step). The position/direction calculator 12 transmits a result of the calculated at least one of the absolute position and the absolute direction of the capsule endoscope 6 to the control unit 13 as described above.

Thereafter, the control unit 13 controls the magnetic field generating device 1 based on at least one of the absolute position and the absolute direction of the capsule endoscope 6 obtained from the position/direction calculator 12 (a magnetic field controlling step). In this case, the control unit 13 controls the magnetic field generating device 1 through a control of the guiding magnetic field controller 19 described above. The guiding magnetic field controller 19 makes the magnetic field generating device 1 form a rotating and guiding magnetic field to be applied to the capsule endoscope 6 so as to guide the capsule endoscope 6 to at least one of the position and the direction instructed by the control unit 13. In this manner, the capsule guiding system 10 can allow a precise guidance of the capsule endoscope 6 within the human body 5 to at least one of a desired position and a desired direction.

In the first embodiment, since the electrode pads 7 and the human body are electrically in contact with each other via the conductive gel bed 4, it is possible to perform a stable human body communication and detect at least one of the position and the direction of the capsule endoscope 6. Besides, since the electrode pads 7 have a function of the human body communication as well as a function of detecting at least one of the position and the direction of the capsule endoscope 6, a configuration can be simple in the first embodiment. Furthermore, since the electrode pads 7 are fixedly arranged on the bed 3, the positional relationship between the magnetic field generating device 1 and the electrode pads 7 is already known and thereby the precision of at least one of the absolute position and the absolute direction of the capsule endoscope 6 can be virtually determined only based on the precision in the detection by the electrodes pads 7 of at least one of the relative position and the relative direction of the capsule endoscope 6, and since the precision in the detection of at least one of the relative position and the relative direction by the electrode pads 7 as described above is high enough, the precision in the detection of at least one of the absolute position and the absolute direction of the capsule endoscope 6 can be enhanced ultimately in the first embodiment. In other words, the detected position/direction of the capsule endoscope 6 corresponds, in coordinates, to the position/direction of the capsule endoscope 6 which is supposed to be controlled by the magnetic field generating device 1. As a result, a guiding control for moving the capsule endoscope 6 can be performed with high precision.

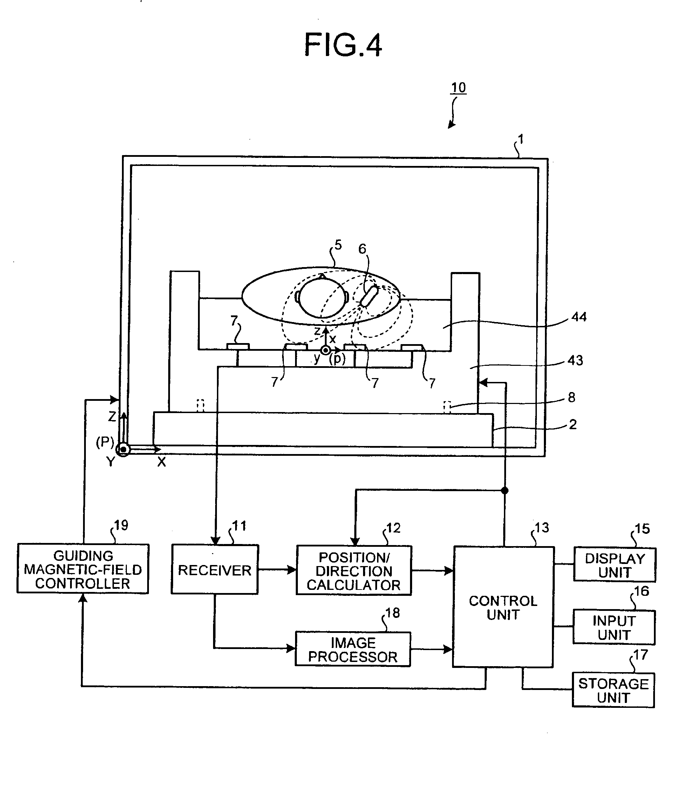

Though the conductive gel bed 4 is provided between the human body 5 and the bed 3 in the first embodiment described above, the present invention is not limited to this and a waterbed may be used instead of the conductive gel bed 4, for example. Besides, as shown in FIG. 4, a bathtub 43 may be provided on the base 2, the electrode pads 7 may be provided inside the bathtub 43, and the bathtub 43 may be filled with a conductive fluid 44 such as fresh water, instead of the conductive gel bed 4. As understood from the fact that a most part of the human body is constituted by moisture contents, water is conductive and has an impedance whose value is close to a human body impedance. Conversely, the impedance of the conductive gel bed 4 and the conductive fluid 44 is preferably close to about 20 ohms which is the impedance of the human body 5. Moreover, to make the impedance of water correspond to the impedance of the human body, a physiologic saline may be used instead of fresh water.

In addition, the bed 3 may be configured to be movable to an X-axis direction and a Z-axis direction except for the Y-axis direction. Besides, though the bed is explained as an example of an object which arranges the human body 5, an object such as a chair having a shape on which the human body is seated and another object having a columnar shape or a wall shape to be used in a way that the human body 5, while standing, leans against the conductive soft part of the human body arranging device may be adopted except for the bed.

### Second embodiment

Next, a second embodiment not forming part of the present invention will be explained. Though all of the electrode pads 7 via the conductive gel bed 4 are treated as a target of the human body communication and the position/direction detection in the first embodiment described above, the human body communication and the position/direction detection in the second embodiment are performed without providing the conductive gel bed 4 and by treating only electrode pad(s) 7 that is/are in contact with the human body 5 on the bed 3 as a detection target.

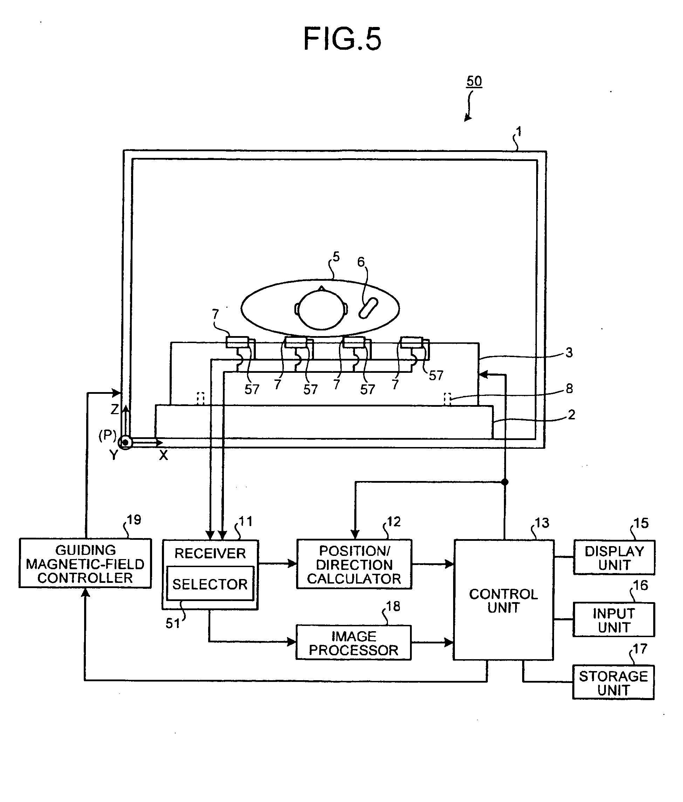

FIG. 5 is a schematic view showing a structure of a capsule guiding system according to the second embodiment. As shown in FIG. 5, a capsule guiding system 50 has a configuration eliminating the conductive gel bed 4 shown in the capsule guiding system 10 and is provided with a plurality of pressure sensors 57 which are respectively in contact with electrode pads 7 or placed in the vicinity of the electrode pads 7 and detect a contact with a human body. Besides, a detection result of each pressure sensor 57 is transmitted to the receiver 11 and a selector 51 provided in the receiver 11 outputs, to the position/direction calculator 12 and the image processor 18, only a detection result of an electrode pad 7 corresponding to a pressure sensor 57 which has detected a pressure not less than a predetermined value, the pressure not less than the predetermined value allowing to assume that the human body is in contact with the corresponding electrode pad 7. Other constituents are the same as those in the first embodiment.

Since the conductive gel bed 4 is not necessary in the second embodiment, it is possible to downsize the capsule guiding system.

Though the pressure sensors 57 are used as a sensor which detects a contact with a human body in the second embodiment described above, the present embodiment is not limited to this and a temperature sensor or a mechanical switch may be used, for example.

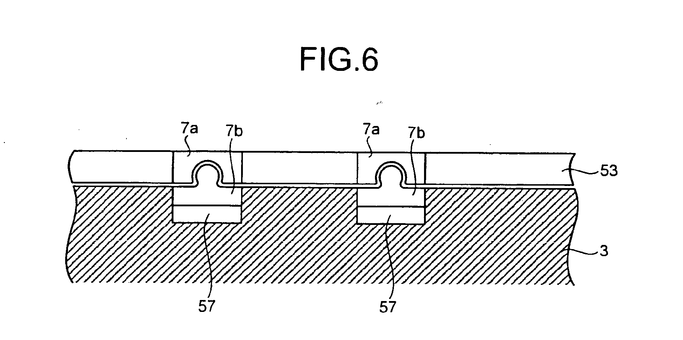

Furthermore, the electrode pad 7 may be separated into an electrode pad 7a whose center has a concave shape and an electrode pad 7b whose center has a convex shape, the concave part and the convex part may be engaged to be joined together like a hook, and a sheet member 53 which has an arrangement in which the electrode pad 7a corresponds to the electrode pad 7b may be used as shown in FIG. 6. By this, the sheet member 53 can be changed, so that a hygienic control and a maintenance can be easily performed when an examination is performed repeatedly.

### Third embodiment

Next, a third embodiment not forming part of the present invention will be explained. In contrast to the configuration of fixedly arranging the electrode pads 7 on the bed 3 in the first and the second embodiments described above, electrode pads are arranged to move in accordance with a movement of a human body in the third embodiment.

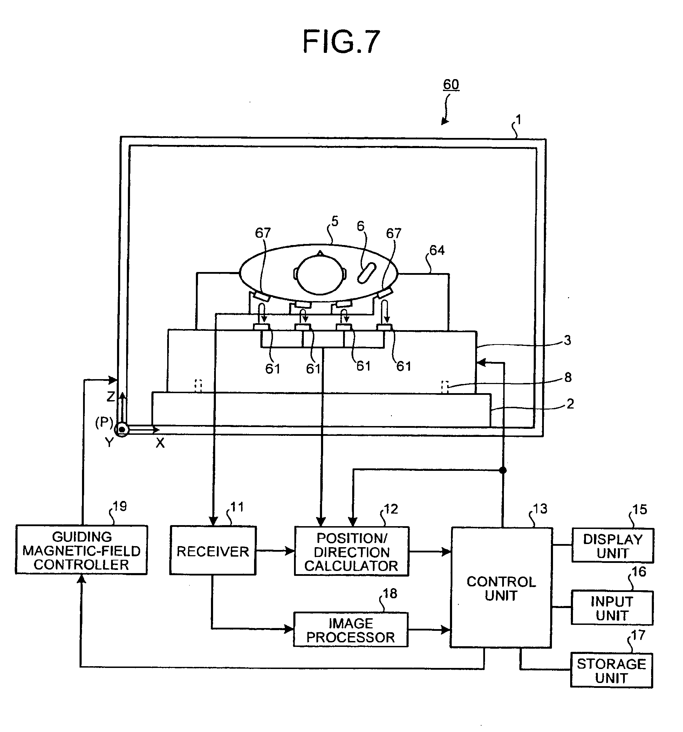

FIG. 7 is a schematic view of a structure of a capsule guiding system according to the third embodiment. A capsule guiding system 60 shown in FIG. 7 is provided with a gel bed 64 (the soft part of the human body arranging device) instead of the conductive gel bed 4 in the capsule guiding system 10, and a plurality of electrode pads 67 arranged like a matrix are attached to a side of the human body 5 on the gel bed 64. Thus, the electrode pads 67 change in position in accordance with a movement of the human body 5. Therefore, sensors 61 which are respectively associated with the electrode pads 67 are provided on a side of the bed 3 of the gel bed 64 or on an upper surface of the bed 3, each sensor 61 being capable of detecting a positional change of the associated electrode pad 67. The sensor 61 is, for example, realized by an ultrasonic sensor and detects a distance from or a positional change of the associated electrode pad 67 by using an echo of the ultrasonic generated by the ultrasonic sensor. The detection result is output to the position/direction calculator 12 and the position/direction calculator 12 corrects the position of the electrode pad 67 based on the position of the electrode pad 67 detected by the sensor 61. Other constituents are the same as those in the first embodiment.

It is possible to perform a stable human body communication and detect the position/direction of the capsule endoscope with high precision in the third embodiment, too.

### Fourth embodiment

Next, a fourth embodiment not forming part of the present invention will be explained. Though the position of an electrode pad is detected from the side of the bed 3 in the third embodiment described above, the positional change of an electrode pad is detected from a side opposite to the bed 3, that is, from an outside in the fourth embodiment.

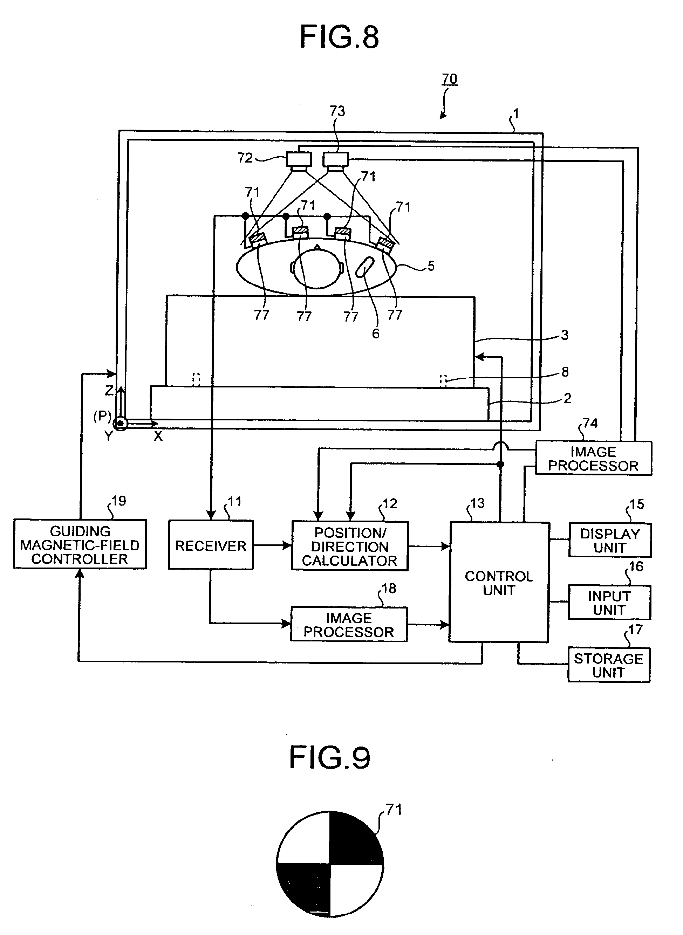

FIG. 8 is a schematic view of a structure of a capsule guiding system 70 according to the fourth embodiment. In FIG. 8, a plurality of electrode pads 77 are provided on a surface of the human body 5 and voltage values detected in the electrode pads 77 are output to the receiver 11. A Marker 71 having a pattern shown in FIG. 9 is attached to an outside surface of each electrode pad 77.

On the other hand, two imaging devices 72 and 73 which image the plurality of markers 71 are arranged at a given distance at an outside of the human body 5, an image processing, performed by an image processor 74, for calculating a three-dimensional position seen from each of the imaging devices 72 and 73 to each marker 71 is performed on the image captured by each of the imaging devices 72 and 73, and a result of the processing is output to the position/direction calculator 12. Since positions of the imaging devices 72 and 73 with respect to the reference position P of the magnetic field generating device 1 are known and fixed, the position/direction calculator 12 can calculate a position of each electrode pad 77 with respect to the reference position P of the magnetic field generating device 1 and can calculate at least one of an absolute position and an absolute direction of the capsule endoscope 6 with respect to the reference position P of the magnetic field generating device 1 based on the three-dimensional position of each electrode pad 77 and on at least one of a relative position and a relative direction, calculated depending on each electrode pad 77, of the capsule endoscope 6.

It is possible to precisely detect at least one of the absolute position and the absolute direction of the capsule endoscope 6 in the fourth embodiment, too.

Though the human body 5 is configured to lie down on the bed 3 in the fourth embodiment described above, the present embodiment is not limited to this and an application to a case where the human body 5 stands on his/her own feet is also available. Besides, an application to another case where the human body 5 is seated on a chair and the like is also available.

Besides, though the markers 71 are provided to obtain three-dimensional positions of the electrode pads 77 respectively through a stereovision of the markers 71, the present embodiment is not limited to this and magnetic sensors such as a resonance coil, an LC marker, an MI (magnetoimpedance) sensor, and an MR (magnetoresistance) sensor are provided instead of the markers 71 and each magnetic sensor may detect a certain guiding magnetic field to detect the three-dimensional position of each electrode pad.

Moreover, an ultrasonic scanner, a three-dimensional scanner using a light, and the like may be used to scan a surface of the human body on a side of the electrode pad, detect a movement of the human body based on a scanned image, and detect or estimate the three-dimensional position of each electrode pad based on the detection result.

Futhermore, a plurality of mechanical displacement gauges may be provided at a tip end of an arm which is not shown, and a displacement of each electrode pad may be converted to a mechanical displacement to detect the three-dimensional position of each electrode pad while the mechanical displacement gauge is pressed onto the electrode pad. Here, the electrode pad may be provided at a tip end of the mechanical displacement gauge. By this, a contact between a human body and an electrode pad becomes stable and the configuration becomes simple.

Though transmitting electrodes of the capsule endoscope 6 are realized by the transmitting electrode 22 which is transparent and provided at the imaging side, and the transmitting electrode 23 having a dome shape part at the opposite side to the transmitting electrode 22 in the embodiments 1 to 4 described above, the present invention is not limited to this, and an arrangement and a pattern of a pair of transmitting electrodes can be arbitrarily determined. For example, a pair of transmitting electrodes may be provided on the spiral protrusion 24, or double spiral protrusions may be provided and a transmitting electrode may be provided to each spiral protrusion. This configuration allows a contact state between the capsule endoscope 6 and the human body 5 to be stable.

Besides, to enhance communication characteristics of the human body communication, ionized water whose impedance is close to the impedance of the human body 5 may be taken at the time of an examination, so that the contact state between the capsule endoscope 6 and the human body 5 can be improved. Furthermore, though the method of causing a rotation of the spiral protrusion is explained as a method of guiding the capsule endoscope 6, the present invention is not limited to this, and an application to a method of using a magnetic gradient to pull and guide the capsule endoscope 6 through a magnetic attraction force is also available.

### INDUSTRIAL APPLICABILITY

As described above, a capsule guiding system according to the present invention is useful for guiding a capsule endoscope inserted in an inside of a human body, and specifically suitable as a capsule guiding system which can allow a precise guidance of a capsule endoscope that performs a human body communication to at least one of a desired position and a desired direction.

## Claims

1. A capsule guiding system (10) comprising:
an electrode pad (7) configured to perform a human body communication with a capsule endoscope (6) and to detect at least one of a position and a direction of the capsule endoscope (6);
a human body arranging device comprising a rigid part (3) and a conductive soft part (4), the electrode pad (7) being fixedly arranged between the conductive soft part (4) and the rigid part (3) so that during use, the electrode pad (7) and the human body (5) are electrically in contact with each other via the conductive soft part (4);
a magnetically guiding device (1) configured to move the capsule endoscope (6);
a calculator (12) configured to calculate at least one of the position and the direction of the capsule endoscope (6) based on a detection value of the electrode pad (7), to add at least one of a position and a direction of the rigid part (3) of the human body arranging device with respect to the magnetically guiding device (1) to at least one of the position and the direction of the capsule endoscope (6), and to calculate at least one of an absolute position and an absolute direction of the capsule endoscope (6) with respect to the magnetically guiding device (1);
a receiver (11) configured to receive voltages induced in the electrode pad (7) and to output them to said calculator (12); and
a control unit (13) configured to control the magnetically guiding device (1) based on at least one of the absolute position and the absolute direction.

2. The capsule guiding system (10) according to claim 1, wherein the conductive soft part (4) of the human body arranging device has an impedance virtually equal to a human body impedance.

3. The capsule guiding system (10) according to claim 1, further comprising a human body moving unit which is configured to relatively move the human body (5) with respect to the magnetically guiding device (1), wherein the calculator (12) is configured to use a value of the relative movement by the human body moving unit to correct at least one of the absolute position and the absolute direction of the capsule endoscope (6).

## Patentansprüche

1. Kapselführungssystem (10) aufweisend:
ein Elektrodenpad (7), welches dazu eingerichtet ist, eine Kommunikation über einen menschlichen Körper mit einem Kapselendoskop (6) auszuführen und zumindest eine Position und eine Richtung des Kapselendoskops (6) zu erfassen;
eine Vorrichtung zum Ausrichten eines menschlichen Körpers aufweisend ein steifes Teil (3) und ein leitendes weiches Teil (4), wobei das Elektrodenpad (7) fest zwischen dem leitenden weichen Teil (4) und dem steifen Teil (3) angeordnet ist, so dass während der Verwendung das Elektrodenpad (7) und der menschliche Körper (5) über das leitende weiche Teil (4) elektrisch miteinander in Verbindung stehen;
eine magnetische Führungsvorrichtung (1), die dazu eingerichtet ist, das Kapselendoskop (6) zu bewegen;
einen Rechner (12), der dazu eingerichtet ist, die Position und/oder die Richtung des Kapselendoskops (6) basierend auf einem Erfassungswert des Elektrodenpads (7) zu berechnen, eine Position und/oder eine Richtung des steifen Teils (3) der Vorrichtung zum Ausrichten des menschlichen Körpers hinsichtlich der magnetischen Führungsvorrichtung (1) zu der Position und/oder der Richtung des Kapselendoskops (6) zu addieren, und eine absolute Position und/oder eine absolute Richtung des Kapselendoskops (6) hinsichtlich der magnetischen Führungsvorrichtung (1) zu berechnen;
einen Empfänger (11), der dazu eingerichtet ist, in das Elektrodenpad (7) induzierte Spannungen zu empfangen und sie zu dem Rechner (12) auszugeben; und
eine Steuerungseinheit (13), die dazu eingerichtet ist, die magnetische Führungsvorrichtung (1) basierend auf der absoluten Position und/oder der absoluten Richtung zu steuern.

2. Kapselführungssystem (10) nach Anspruch 1, bei dem das leitende weiche Teil (4) der Vorrichtung zum Ausrichten des menschlichen Körpers eine Impedanz aufweist, die nahezu gleich einer Impedanz des menschlichen Körpers ist.

3. Kapselführungssystem (10) nach Anspruch 1, ferner aufweisend eine Einheit zum Bewegen des menschlichen Körpers, die dazu eingerichtet ist, den menschlichen Körper (5) relativ zu der magnetischen Führungsvorrichtung (1) zu bewegen, wobei der Rechner (12) dazu eingerichtet ist, einen Wert der relativen Bewegung durch die Einheit zum Bewegen des menschlichen Körpers zum Korrigieren der absoluten Position und/oder der absoluten Richtung des Kapselendoskops (6) zu verwenden.

## Revendications

1. Système de guidage de capsule (10) comprenant :
une électrode (7) configurée pour réaliser une communication d'un corps humain avec une capsule endoscopique (6) et pour détecter au moins l'une parmi une position et une direction de la capsule endoscopique (6) ;
un dispositif de disposition de corps humain comprenant une partie rigide (3) et une partie souple conductrice (4), l'électrode (7) étant disposée de manière fixe entre la partie souple conductrice (4) et la partie rigide (3) de sorte que pendant l'utilisation, l'électrode (7) et le corps humain (5) sont en contact électrique l'un avec l'autre via la partie souple conductrice (4) ;
un dispositif de guidage magnétique (1) configuré pour déplacer la capsule endoscopique (6) ;
un calculateur (12) configuré pour calculer au moins l'une parmi la position et la direction de la capsule endoscopique (6) sur la base d'une valeur de détection d'électrode (7), pour ajouter au moins l'une parmi une position et une direction de la partie rigide (3) du dispositif de disposition de corps humain par rapport au dispositif de guidage magnétique (1) à au moins l'une parmi la position et la direction de la capsule endoscopique (6), et pour calculer au moins l'une parmi une position absolue et une direction absolue de la capsule endoscopique (6) par rapport au dispositif de guidage magnétique (1) ;
un récepteur (11) configuré pour recevoir des tensions induites dans l'électrode (7) et pour les délivrer en sortie vers ledit calculateur (12) ; et
une unité de commande (13) configurée pour commander le dispositif de guidage magnétique (1) sur la base d'au moins l'une parmi la position absolue et la direction absolue.

2. Système de guidage de capsule (10) selon la revendication 1, dans lequel la partie souple conductrice (4) du dispositif de disposition de corps humain présente une impédance virtuellement égale à une impédance de corps humain.

3. Système de guidage de capsule (10) selon la revendication 1, comprenant une unité de déplacement de corps humain qui est configurée pour déplacer le corps humain (5) de manière relative par rapport au dispositif de guidage magnétique (1), dans lequel le calculateur (12) est configuré pour utiliser une valeur du mouvement relatif par l'unité de déplacement de corps humain pour corriger au moins l'une parmi la position absolue et la direction absolue de la capsule endoscopique (6).
